# EUROPEAN PATENT SPECIFICATION

(11) **EP 2 987 853 B1**
(45) Date of publication and mention of the grant of the patent: **31.05.2017**
(21) Application number: 14785480.6
(22) Date of filing: 16.04.2014
(51) Int. Cl.: C12N 1/20, C12N 15/09, C12R 1/225

(54) **BACTERIUM BELONGING TO GENUS LACTOBACILLUS**
BAKTERIUM DER GATTUNG LACTOBACILLUS
BACTERIE APPARTENANT AU GENRE LACTOBACILLUS

(30) Priority: 17.04.2013 JP 2013086575
(43) Date of publication of application: 24.02.2016
(73) Proprietor: Suntory Holdings Limited, Osaka-shi, Osaka 530-8203 (JP)
(72) Inventor: FUKUSHIMA, Eiji, Kanagawa 211-0067 (JP); OKADA, Sanae, Tokyo 156-8502 (JP)
(74) Representative: Vossius & Partner Patentanwälte Rechtsanwälte mbB
(86) International application number: PCT/JP2014/060811
(87) International publication number: WO 2014/171477

(56) References cited:
- JP-A- H07 250 649
- JP-A- 2004 073 178
- JP-A- 2007 135 596
- JP-A- 2007 284 360
- JP-A- 2008 179 595
- JP-A- 2011 217 715
- TOSHIHIRO MAEKAWA ET AL.: 'Lactobacillus pentosus S- PT 84-kabu ni yoru Kohiman Koka' JAPAN SOCIETY FOR BIOSCIENCE, BIOTECHNOLOGY, AND AGROCHEMISTRY TAIKAI KOEN YOSHISHU vol. 2011, 2011, page 67
- SOGAWA M. ET AL.: 'Awa (Tokushima) lactate- fermented tea as well as green tea enhance the effect of diet restriction on obesity in rats' J. MED. INVEST. vol. 56, 2009, pages 42 - 8, XP055286736
- YUEI SHU ET AL.: 'Rat ni Okeru Nyusankin no Shokugo Ko Triacylglycerol Kessho Kaizen Sayo' JAPAN SOCIETY FOR BIOSCIENCE, BIOTECHNOLOGY, AND AGROCHEMISTRY TAIKAI KOEN YOSHISHU vol. 2011, 2011, page 67

## Description

### TECHNICAL FIELD

The present invention relates to a bacterium belonging to the genus *Lactobacillus.* More specifically, the present invention relates to novel *Lactobacillus pentosus.*

### BACKGROUND ART

Some of lactic acid bacteria and Bifidobacteria have excellent physiological activities such as an intestine regulating activity and immunostimulating activity, and have been used in various applications depending upon the properties of the bacterial species. Among them, recently, studies on dieting effects by taking these bacteria have been progressed, and many reports have been made.

For example, Patent Publication 1 reports that *Lactobacillus rhamnosus* ATCC53103 strain degrades a lipid (triacyl glycerol) which is causative of obesity, thereby blocking its absorption into the body. In addition, it has been known that *L. brevis* KB290, which is one kind of vegetable lactic acid bacteria, reaches to the intestines in a live state, thereby showing excellent intestinal viable rates and intestinal tract survivability (however, the number of bacteria excreted is smaller than the number of ingested bacteria) (see Non-Patent Publication 1). Also, Non-Patent Publication 2 has reported that *Lactobacillus acidophilus* L-92 strain is collected from feces in an amount 93% of the number of ingested bacteria, so that the strain has excellent intestinal tract survivability, and Non-Patent Publication 3 has reported that the survivability of *L. gasseri* SBT2055 in the intestinal tract is examined, and 100 g of a fermented milk containing 1 × 10⁶ to 5 × 10⁶ cfu/g of the bacteria is administered, and as a result, the bacteria are detected from feces maximally at 1 × 10⁵ cfu/g or so.

On the other hand, as to the Bifidobacteria, it has been reported that *Bifidobacterium animalis subspecies lactis* GCL2505 strain not only has intestinal tract survivability in which the strain reaches to the intestines in a live state after the oral ingestion but also shows remarkable proliferation ability within the intestinal tract (see, Patent Publication 2). Non-Patent Publication 4 has reported that when *B. animalis ssp. lactis* DN-173 010 is administered to adults, 20% or so of the DN-173 010 is detected from stools, relative to the number of bacteria ingested.

### RELATED ART REFERENCES

### PATENT PUBLICATIONS

Patent Publication 1: Japanese Patent Laid-Open No. 2011-206057
Patent Publication 2: Japanese Patent Laid-Open No. 2011-172506

### NON-PATENT PUBLICATIONS

Non-Patent Publication 1: *"*Physiological Function of Lactic Acid Bacteria for Human Health," August 31, 2007, CMC Publishing CO., LTD., 160-162
Non-Patent Publication 2: Japanese Journal of Lactic Acid Bacteria, 2001, 12, "Isolation and characterization of a Lactobacillus acidophilus strain L92 that can survive in in human gastrointestinal tract," 28-35
Non-Patent Publication 3: Microbiol. Immunol., 2006, 50, "Monitoring and survival of Lactobacillus gasseri SBT2055 in the human intestinal tract.," 867-870
Non-Patent Publication 4*:* J. Mol. Microbiol. Biotechnol., 2008,14, "Survival of Bifidobacterium animalis DN-173 010 in the faecal microbiota after administration in lyophilized form or in fermented product - a randomized study in healthy adults," 128-136

### SUMMARY OF THE INVENTION

### PROBLEMS TO BE SOLVED BY THE INVENTION

Conventionally, various probiotics are examined for survivability in the intestinal tract as described above. However, as to lactic acid bacteria, it is only reported that the number of bacteria surviving in the intestinal tract is of the same level or lower than the number of bacteria ingested, or the number of bacteria surviving in the intestinal tract is not measured.

In addition, since fat absorption mainly takes place in the small intestines, even if *Bifidobacterium* which is usually proliferated in the large intestine shows proliferation ability in the intestinal tract, the blocking of fat absorption is not sufficient. Further, the lactic acid bacteria are known to act upstream the large intestine, but reports on the bacterial species showing proliferation ability in the intestinal tract have not yet been made.

An object of the present invention is to provide lactic acid bacteria showing proliferation ability in the intestinal tract.

### MEANS TO SOLVE THE PROBLEMS

The present invention relates to a *Lactobacillus pentosus* TUA4337L strain (accession number: NITE BP-1479), characterized in that the strain has proliferation ability in the intestinal tract.

### EFFECTS OF THE INVENTION

The lactic acid bacteria of the present invention exhibit excellent effects of proliferating in the intestinal tract. In addition, when the lactic acid bacteria of the present invention are ingested, some excellent effects that physiological activity of the bacterial cells is enhanced such as the lactic acid bacteria survive in the intestinal tract and proliferate, thereby continuing the effects of blocking fat absorption are exhibited, which in turn result in obtaining highly dieting effects.

### BRIEF DESCRIPTION OF THE DRAWINGS

[FIG. 1] FIG. 1 is a graph showing the results of screening in artificial intestinal solutions.
[FIG. 2] FIG. 2 is a graph showing the transition of weight gains, wherein "*" marks in the figure show that there is a significant difference (p < 0.05) based on the high-fat diet group.
[FIG. 3] FIG. 3 is a graph showing the amount of triglyceride in sera, wherein "*" marks in the figure show that there is a significant difference (p < 0.05) based on the high-fat diet group.

### MODES FOR CARRYING OUT THE INVENTION

The lactic acid bacteria of the present invention are a *Lactobacillus pentosus* TUA4337L strain, characterized in that the strain has proliferation ability in the intestinal tract. Here, the phrase "has proliferation ability in the intestinal tract" or "proliferating in the intestinal tract" as used herein means that the strain after having survived in the intestinal tract proliferates in the small intestines and/or the large intestine, and preferably the small intestines, and the degree of proliferation ability can be evaluated as "being proliferative" in a case where the numerical value is ten times or more of the OD₆₆₀ at inoculation when the strain is cultured in an artificial intestinal solution at 37°C for 6 hours.

The present inventors have examined proliferation ability of about 480 kinds of lactic acid bacteria owned by the present inventors in artificial intestinal solutions, and have administered suspensions of the bacteria belonging to *Lactobacillus pentosus* selected therefrom to animals. As a result, the present inventors have found out that the *Lactobacillus pentosus* TUA4337L strain is significantly larger in the number of bacteria excreted than the number of bacteria administered. The present invention has been perfected thereby.

The *Lactobacillus pentosus* TUA4337L strain is deposited at Patent Microorganisms Depositary, National Institute of Technology and Evaluation, Incorporated Administrative Agency (2-5-8 Kazusakamatari, Kisarazu-shi, Chiba-ken, Japan) with the identification reference of NRIC 0883, under the accession number of NITE BP-1479 dated with an international deposition date of December 10, 2012. The *Lactobacillus pentosus* TUA4337L strain is hereinafter simply referred to as TUA4337L strain.

The bacteriological characteristics of the TUA4337L strain are shown in Tables 1 and 2. The sugar assimilation activity of Table 2 is the results of measurement using a bacteria identification kit API 50CH (BIOMETRIEUX). Here, "+" means an assimilated sugar, and "-" means an unassimilated sugar in Table 2.

[Table 1]

**Table 1**

| Bacterial Morphology | *Bacillus* |
|---|---|
| Gram Staining | Positive |
| Mobility | Absent |
| Spore | Absent |
| End Spore | Absent |
| Catalase Reaction | Negative |
| Growth at 15°C | ○ |
| Growth at 40°C | ○ |
| Aerobic Growth | ○ |
| Anaerobic Growth | ○ |
| pH at Growth | 3.0-12.5 |

[Table 2]

**Table 2**

| Sugar Assimilation Activity | | Sugar Assimilation Activity | | Sugar Assimilation Activity | |
|---|---|---|---|---|---|
| Glycerol | + | D-Mannitol | + | D-Raffinose | + |
| Erythritol | - | D-Sorbitol | + | Starch | - |
| D-Arabinose | - | Methyl-αD-Glucopyranoside | + | Glycogen | - |
| L-Arabinose | + | N-Acetylglucosamine | + | Xylitol | - |
| D-Ribose | + | Amygdalin | + | Gentiobiose | + |
| D-Xylose | + | Arbutin | + | D-Turanose | + |
| L-Xylose | - | Ferric Citrate-Aesculin | + | D-Lixose | - |
| D-Adonitol | - | Salicin | + | D-Tagatose | - |
| Methyl-(βD-xylopyranoside | - | D-Cellobiose | + | D-Fucose | - |
| D-Galactose | + | D-Maltose | + | L-Fucose | - |
| D-Glucose | + | D-Lactose | + | D-Arabitol | - |
| D-Fructose | + | D-Melibiose | + | L-Arabitol | - |
| D-Mannose | + | D-Sucrose | + | Gluconate | + |
| L-Sorbose | - | D-Trehalose | + | 2-Ketogluconate | - |
| Dulcitol | - | Inulin | - | 5-Ketogluconate | - |
| Inositol | - | D-Melezitose | - | | |

As described in detail in Examples set forth below, the TUA4337L strain has the characteristics of increasing the number of bacteria excreted as compared to the number of bacteria ingested, in other words, having proliferation ability in the intestinal tract. In addition, as the proliferation ability in the intestinal tract, the number of bacteria after 6-hour culture in an artificial intestinal solution at 37°C is preferably 10 times or more, more preferably 15 times or more, even more preferably 20 times or more, and still even more preferably 25 times or more, of the number of bacteria at the beginning of culture of the bacteria used as a standard.

In addition, the sequence of *recA* gene (SEQ ID NO: 1) decoded from DNA extracted from the TUA4337L strain has 99% homology to the sequence of *recA* gene of *Lactobacillus pentosus* IG1 strain, Here, the homology as used herein is shown as a degree of similarity by scores using, for example, a search program BLAST using the algorithm developed by Altschul et al. (The Journal of Molecular Biology, 215, 403-410 (1990)).

The medium for culturing the TUA4337L strain is not particularly limited, and the medium includes media containing ordinary carbon sources, nitrogen sources, inorganic salts, organic nutrients and the like. In addition, the culture with an agar medium or a liquid medium can be performed. The culture temperature is preferably from 10° to 45°C, more preferably from 15° to 42°C, even more preferably from 28° to 38°C, and even more preferably from 35° to 37°C, and a proliferative pH is preferably a pH of from 3.0 to 12.5, and more preferably a pH of from 3.5 to 12.0.

The bacterial strain of the present invention includes lactic acid bacteria themselves, including viable bacteria and dead bacteria, and in various forms such as lactic acid bacteria inclusions and processed cells of lactic acid bacteria. The viable bacteria can be obtained from lactic acid bacteria inclusions such as a culture medium containing lactic acid bacteria. The dead bacteria can be obtained, for example, by subjecting viable bacteria to heating, ultraviolet irradiation, formalin treatment, an acid treatment or the like. The resulting viable bacteria or dead bacteria can be further produced into processed cells by subjecting the bacteria to grinding, crushing, or the like. Here, the lactic acid bacteria in each of the above forms are preferably viable bacteria from the viewpoint of fully exhibiting the effects of proliferating in the intestinal tract, and dead bacteria may be admixed therewith.

The above lactic acid bacteria include, for example, viable bacteria, wet bacteria, dry bacteria, and the like. The above lactic acid bacteria inclusions include, for example, suspensions of lactic acid bacteria, cultured cells of lactic acid bacteria (including bacterial cells, supernatant, and medium ingredients), and cultured media containing lactic acid bacteria (obtained by removing solid contents from the cultured cells of bacteria). In addition, the above processed cells of lactic acid bacteria include, for example, ground cells, crushed cells, liquefied cells (extracts etc.), concentrates, paste-like cells, dried cells (spray-dried cells, freeze-dried cells, vacuum-dried cells, drum-dried cells, etc.), diluted cells, and the like.

### EXAMPLES

The present invention will be specifically described hereinbelow by the Examples, without intending to limit the scope of the present invention to the following Examples.

### Example 1 < Screening Using Proliferation Ability in Artificial Intestinal Solution as Index >

Among the lactic acid bacteria owned by the present inventors, the proliferation ability in an artificial intestinal solution was evaluated for about 480 strains which were mainly vegetable lactic acid bacteria (including JCM strains).

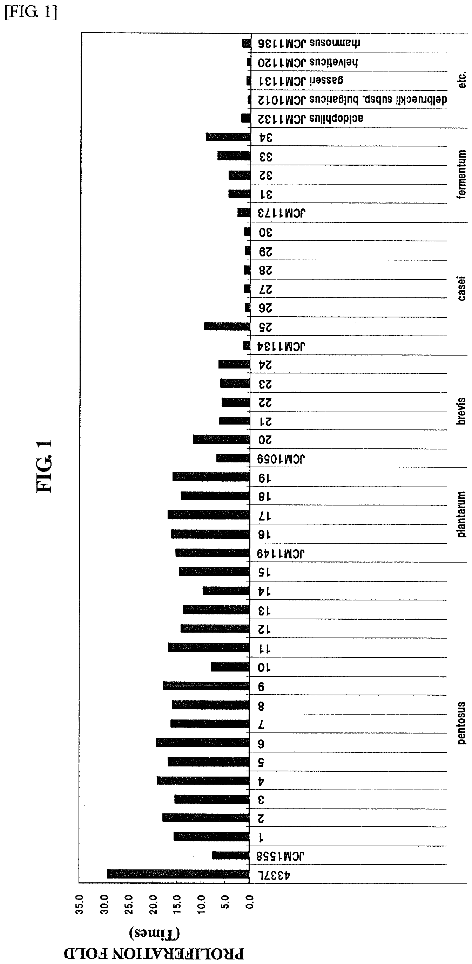

Concretely, first, each of the lactic acid bacteria was inoculated from a glycerol stock to an MRS medium (Difco Laboratories) (10mL) in an amount of 1 v/v% each, and the bacterial cells were cultured at 35°C for 16 to 17 hours. Next, OD₆₆₀ of each culture medium (absorbance at 660 nm) was measured with a spectrophotometer UV-1600 (Shimadzu Corporation), and a 100 µL solution prepared with the MRS medium so that OD₆₆₀ of each culture medium would be 10 was inoculated to an artificial intestinal solution (10mL) of the composition shown hereinbelow. Thereafter, the bacterial cells were cultured at 37°C for 6 hours while gently shaking, and OD₆₆₀ was then measured to obtain a proliferation fold (OD₆₆₀ after 6 hours/OD₆₆₀ at inoculation). The representative screening results are shown in Table 3 and FIG. 1.

**< Artificial Intestinal Solution (pH 6.45) >**

| | |
|---|---|
| MRS Medium | 9 mL |
| 10 w/v% bile acid (Wako Pure Chemical Industries, Ltd.) solution | 1 mL |
| 1 w/v% Pancreatin (from Porcine: SIGMA) | 100 µL |

Here, the bile acid solution and the pancreatin solution, which were made sterile by treating the solution with a 0.22 µm filter (PVDF membrane, manufactured by Millipore), were used.

[Table 3]

**Table 3**

| Genera, Species | Strain | OD₆₆₀ after 6 hours | Proliferation Fold (times) (OD₆₆₀ after 6 hours/ OD₆₆₀ at Inoculation) |
|---|---|---|---|
| | TUA4337L (Present Invention) | 2.93 | 29.3 |
| | JCM1558 | 0.73 | 7.3 |
| | 1 | 1.55 | 15.5 |
| | 2 | 1.79 | 17.9 |
| | 3 | 1.54 | 15.4 |
| | 4 | 1.90 | 19.0 |
| | 5 | 1.68 | 16.8 |
| *Lactobacillus pentosus* | 6 | 1.93 | 19.3 |
| | 7 | 1.60 | 16.0 |
| | 8 | 1.60 | 16.0 |
| | 9 | 1.78 | 17.8 |
| | 10 | 0.78 | 7.8 |
| | 11 | 1.68 | 16.8 |
| | 12 | 1.42 | 14.2 |
| | 13 | 1.37 | 13.7 |
| | 14 | 0.96 | 9.6 |
| | 15 | 1.46 | 14.6 |
| *Lactobacillus plantarum* | JCM1149 | 1.53 | 15.3 |
| | 16 | 1.63 | 16.3 |
| | 17 | 1.70 | 17.0 |
| | 18 | 1.42 | 14.2 |
| | 19 | 1.59 | 15.9 |
| *Lactobacillus brevis* | JCM1059 | 0.68 | 6.8 |
| | 20 | 1.18 | 11.8 |
| | 21 | 0.63 | 6.3 |
| | 22 | 0.58 | 5.8 |
| | 23 | 0.61 | 6.1 |
| | 24 | 0.65 | 6.5 |
| *Lactobacillus casei* | JCM1134 | 0.14 | 1.4 |
| | 25 | 0.94 | 9.4 |
| | 26 | 0.10 | 1.0 |
| | 27 | 0.12 | 1.2 |
| | 28 | 0.12 | 1.2 |
| | 29 | 0.11 | 1.1 |
| | 30 | 0.13 | 1.3 |
| *Lactobacillus fermentum* | JCM1173 | 0.27 | 2.7 |
| | 31 | 0.45 | 4.5 |
| | 32 | 0.45 | 4.5 |
| | 33 | 0.69 | 6.9 |
| | 34 | 0.93 | 9.3 |
| *Lactobacillus acidophilus* JCM1132 | | 0.19 | 1.9 |
| *Lactobacillus delbrueckii subsp. bulgaricus* JCM1012 | | 0.06 | 0.6 |
| *Lactobacillus gasseri* JCM1131 | | 0.08 | 0.8 |
| *Lactobacillus helveticus* JCM1120 | | 0.07 | 0.7 |
| *Lactobacillus rhamnosus* JCM1136 | | 0.17 | 1.7 |

As a result, it can be seen that the proliferation folds are more likely to be high in *Lactobacillus pentosus* and *Lactobacillus plantarum,* among which the *Lactobacillus pentosus* TUA4337L strain has an especially high proliferation fold and excellent proliferation ability in the intestinal tract.

### Example 2 < Evaluation of in vivo Proliferation Ability in Intestinal Tract >

Mice subjected to a high-fat diet *ad libitum* were administered with the TUA4337L strain prepared as follows, and the number of bacteria excreted was quantified. Concretely, C57BL/6J mice (10-week old, male) were administered in a single dose with about 1.0 × 10⁹ lactic acid bacteria cells (corresponding to 250 µL of bacterial cell suspension) at 10 o'clock in the morning (n=5), using the administration sample prepared as follows.

### < Preparation of Administration Samples (Viable Bacteria-Containing Samples) >

[1] inoculating TUA4337L strain from a glycerol stock to an MRS medium (30 mL) in an amount of 1 v/v%;
[2] culturing bacterial cells (35°C, 20 hours);
[3] centrifuging the culture medium (8,000 rpm, 5 min) to remove the supernatant, and suspending in 30 mL of PBS(-);
[4] centrifuging the suspension of [3] (8,000 rpm, 5 min) to remove the supernatant, and re-suspending in 5 mL of PBS(-);
[5] counting the number of bacteria with a microscope; and
[6] dispensing a solution containing 20,000,000,000 cells to a 15 mL centrifugation tube, centrifuging (8,000 rpm, 5 min) the solution to remove supernatant, and thereafter suspending in 5 mL of a liquid feed (high-fat diet 60 kcal %FAT: Research Diet) to prepare a bacterial cell suspension (liquid feed was prepared with PBS(-)).

Thereafter, all the stools of two-day portions were collected in 4 divided times (the afternoon of the day the test started, the morning and the afternoon of the following day, and the morning of the day after the following day), the number of bacteria for all the stools was quantified by the following method, and the rate of increase in TUA4337L strain in the intestine in each of mice (the number of bacteria for all the stools/the number of administered bacteria) was calculated. The results are shown in Table 4.

### < Method for Measuring the Number of Bacteria According to Real-Time PCR >

[1] adding 1 mL of PBS(-) to 100 mg of stools (wet weight basis), and then disrupting the stools with a spatula;
[2] collecting a 100 mg portion of the stools to an Eppendorf tube (registered trademark), centrifuging (15,000 rpm, 5 min) the stools to remove supernatant, and suspending the precipitation in 1 mL of PBS(-) (the procedures of centrifuging to suspending being repeated twice);
[3] removing supernatant from the suspension of [2], and thereafter extracting DNA from the suspension with a kit (QIAamp DNA Stool Mini Kit: QIAGEN)
   (the cell disruption being carried out by repeating the procedures three times of adding 300 mg of glass beads (150 to 212 µm: SIGMA), 300 µL of phenol/chloroform/isoamyl alcohol (25:24:1), and 900 µL of buffer ASL (reagents in the kit) to the stools, centrifuging the mixture with MULTI-BEADS SHOCKER MB-200 (YASUI KIKAI) at 3,000 rpm for 1 minute, and allowing to stand on ice for 1 minute); and
[4] quantifying the lactic acid bacteria in the contents of the intestinal tract according to real-time PCR under the conditions shown hereinbelow: ((Conditions for Real-Time PCR))
   (1) Ten microliters of SYBR Premix Ex Taq II (Takara Bio), 0.8 µL of each primer (10 µM), 0.4 µL of ROX reference Dye II, 6 µL of sterile water, and 2 µL of a DNA solution are mixed, to prepare a liquid reaction mixture for PCR. As primers, the following primers specifically detecting 16S rDNA of *Lactobacillus pentosus* and *Lactobacillus plantarum* are used (the 16S rDNA sequences of *Lactobacillus pentosus* and *Lactobacillus plantarum* being 100% identical).
      primer 1: 5'-GCAAGTCGAACGAACTCTGGTATT-3' (SEQ ID NO: 2)
      primer 2: 5'-CGGACCATGCGGTCCAA-3' (SEQ ID NO: 3)
   (2) PCR is performed with 7500 Real Time PCR System (Applied Biosystems), comprising, subsequent to a treatment at 95°C for 30 seconds, carrying out a total of 60 cycles of reactions, wherein one cycle consists of 95°C for 5 seconds and 60°C for 34 seconds. The copy number per one gram of the contents of intestinal tract is obtained from the fluorescent intensity obtained, a total amount of contents of the intestinal tract, and the dilution folds.
   (3) Separately, the copy number of 16S rDNA per one cell is obtained, and the copy number is converted to the number of bacteria. Here, it is confirmed in the mice not administered with the lactic acid bacteria that both *Lactobacillus pentosus* and *Lactobacillus plantarum* are not detected according to the above real-time PCR.

[Table 4]

**Table 4**

| Individual No. | Number of Bacteria of TUA4337L in Stools (cells) | Increased Rate (Number of Bacteria per Entire Stools/Number of Bacteria Administered) |
|---|---|---|
| 1 | 3.6 × 10⁹ | 3.6 |
| 2 | 1.9 × 10⁹ | 1.9 |
| 3 | 2.6 × 10⁹ | 2.6 |
| 4 | 1.6 × 10⁹ | 1.6 |
| 5 | 1.4 × 10⁹ | 1.4 |
| Mean | 2.2 × 10⁹ | 2.2 |

As a result, it could be seen that the number of bacteria in the excretion of the *Lactobacillus pentosus* TUA4337L strain is larger than the number of bacteria ingested. It was suggested from these findings that the *Lactobacillus pentosus* strain passed through the stomach in a viable state, proliferated in the intestinal tract, and excreted.

### Example 3 < Effects of Blocking Weight Gains >

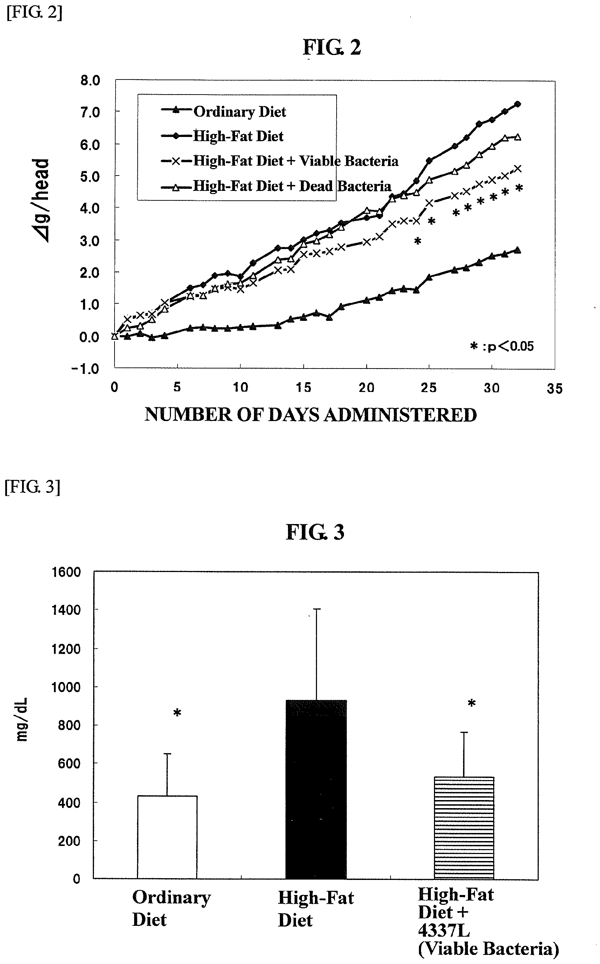

C57BL/6J mice (8-week-old, male) were grouped into four groups of an ordinary diet group, a high-fat diet group, a high fat diet + viable bacteria group, and a high-fat diet + dead bacteria group (n = 10 each), and each of the groups was continuously given with the diets as shown in the following Table 5 for 32 days, and the body weights were measured daily and a mean was calculated. The transition in the mean is shown in FIG. 2. Here, intergroup comparisons were conducted using a t-test with a significant level of 0.05.

Concretely, as to diet, each group of Table 5 was given with each solid feed *ad libitum.* The high-fat diet + viable bacteria group was administered with an administration sample prepared in the same manner as in Example 2. The high-fat diet + dead bacteria group was administered with an administration sample prepared as follows so that the lactic acid bacteria would be contained in an amount of about 1,000,000,000 cells per day. On the other hand, the ordinary diet group was administered with 250 µL of PBS(-) not containing the lactic acid bacteria, and the high-fat diet group was administered with 250 µL of a liquid feed not containing the lactic acid bacteria.

[Table 5]

**Table 5**

| Group | Diet | |
|---|---|---|
| | Solid Diet | Lactic Acid Bacteria Administered |
| Ordinary Diet Group | 10 kcal %FAT | - |
| High-Fat Diet Group | 60 kcal %FAT | - |
| High-Fat Diet + Viable Bacteria Group | 60 kcal %FAT | TUA4337L Viable Bacteria |
| High-Fat Diet + Dead Bacteria Group | 60 kcal %FAT | TUA4337L Dead Bacteria |

| | | |
|---|---|---|
| * 10 kcal %FAT (Research Diet) 60 kcal %FAT (Research Diet) | | |

### < Preparation of Administration Samples (Dead Bacteria-Containing Samples) >

[1] inoculating TUA4337L strain in an amount of 1 v/v % from a glycerol stock to an MRS medium (30 mL);
[2] culturing the bacterial cells (35°C for 20 hours);
[3] centrifuging the culture medium (8,000 rpm, 5 min) to remove supernatant, and thereafter suspending in 30 mL of PBS(-);
[4] centrifuging the suspension of [3] (8,000 rpm, 5 min) to remove supernatant, and thereafter re-suspending in 5 mL of PBS(-);
[5] counting the number of bacteria with a microscope;
[6] dispensing a solution containing 20,000,000,000 cells to a 15 mL centrifugation tube, centrifuging the solution (8,000 rpm, 5 min) to remove supernatant, thereafter adding 5 mL of an artificial gastric fluid (125 mM NaCl, 7 mM KCl, pH 1.0) thereto, stirring the mixture, and allowing to stand for 60 minutes; and
[7] centrifuging the solution of [6] (8,000 rpm, 5 min) to remove supernatant, and thereafter suspending in 5 mL of a liquid feed (60 kcal %FAT) to prepare a bacterial cell suspension.

As a result, the group administered with the TUA4337L viable bacteria showed a significant effect of blocking weight gain, as compared to the control (the high-fat diet group). Also, the administration of viable bacteria was more effective than the administration of dead bacteria. It is considered that the *Lactobacillus pentosus* TUA4337L strain proliferated in the intestinal tract, thereby effectively influencing the host.

### Example 4 <Effects of Blocking Fat Absorption >

The group constituents of the ordinary diet group, the high-fat diet group, and the high-fat diet + viable bacteria group in Example 3 (n = 12 each) were each continued to give the same contents of diets as in Example 3 for 2 weeks. Thereafter, the groups were fasted overnight, and administered with an olive oil (nacalai tesque) (5 mL/kg), and further dissected after 3 hours to collect sera from vena cava. The triglyceride (TG) in sera was measured with Triglyceride E-Test Wako (Wako Pure Chemicals Industries, Ltd.). The results are shown in FIG. 3. Here, the intergroup comparisons were conducted by a significance difference judgment by a t-test with a significant level of 0.05.

As a result, the high-fat diet group was found to show the clear likeliness of increasing the TG in blood as compared to the ordinary diet group. Therefore, it is considered that if a high-fat diet is continued to be ingested, a body would more easily absorb a fat. In addition, the group administered with TUA4337L viable bacteria was found to have blocking of increase in TG in blood, as compared to the control (high-fat diet group). Therefore, one of the mechanisms of the effects of blocking weight gains is considered to be blocking of fat absorption, which was effective even after one day from the administration of the TUA4337L viable bacteria, so that it is considered to exhibit effects continuously.

### INDUSTRIAL APPLICABILITY

Since the lactic acid bacteria of the present invention have proliferation ability in the intestinal tract, when ingested in the body, the lactic acid bacteria survive to the intestinal tract and proliferate, whereby the fat absorption can be continuously blocked, and the weight gains can be effectively blocked, so that the lactic acid bacteria can be suitably used for the purposes of dieting effects.

### SEQUENCE FREE TEXT

SEQ ID NO: 1 of the Sequence Listing is a nucleotide sequence of *recA* of *Lactobacillus pentosus* TUA4337L.
SEQ ID NO: 2 of the Sequence Listing is a nucleotide sequence of a *Lactobacillus pentosus*/*plantarum-*specific primer.
SEQ ID NO: 3 of the Sequence Listing is a nucleotide sequence of a *Lactobacillus pentosus*/*plautarum-*specific primer.

## Claims

1. A *Lactobacillus pentosus* TUA4337L deposited under accession number NITE BP-1479, **characterized in that** the strain has proliferation ability in the intestinal tract.

## Patentansprüche

1. *Lactobacillus pentosus* TUA4337L, der unter der Hinterlegungsnummer NITE BP-1479 hinterlegt ist, **dadurch gekennzeichnet, dass** der Stamm im Darmtrakt Proliferationsfähigkeit aufweist.

## Revendications

1. *Lactobacillus pentosus* TUA4337L, déposé sous le numéro d'accession NITE BP-1479, **caractérisé en ce que** la souche a la capacité de prolifération dans le tractus intestinal.


### SEQUENCE LISTING

<110> SUNTORY HOLDINGS LIMITED
<120> Bacterium belonging to genus Lactobacillus
<130> Y2629 EP S3
<140> EP 14785480.6
   <141> 2014-04-16
<150> JP 2013-86575
   <151> 2013-04-17
<160> 3
<170> PatentIn version Ver. 3.3
<210> 1
   <211> 535
   <212> DNA
   <213> Lactobacillus pentosus 4337L
<220>
   <223> a gene encoding recA of Lactobacillus pentosus 4337L
<400> 1
   gcgattatgc ggatgggtga cgctgcccag acgaccattt caacaatttc cagcgggtca 60
   ctagccttag atgacgcatt aggcgttggt ggttacccac gtggccgaat cgttgaaatt 120
   tatggccctg aaagttccgg taaaacgacc gttgcactac acgcggtcgc tgaagttcaa 180
   aagcaaggcg ggacggccgc ctatatcgat gctgaaaacg ccttggatcc ggtttacgcg 240
   gaacatttag gtgtcaacat tgatgatttg ttactttcac aaccagatac tggtgaacaa 300
   ggtcttgaaa tcgcggatgc tttagtttcc agtggcgcgg ttgatatctt agttgtcgat 360
   tcagttgcgg cgttagtacc acgggccgaa attgaaggtg aaatgggtga cgcccacgtt 420
   ggnttacaag cccggttaat gncacaagcg ttgcggaagt tatccgggac tttgaacaag 480
   acaaagacca tcgcactatt tattaaccaa attcgtgaaa aagttggcgt gatgt 535
<210> 2
   <211> 24
   <212> DNA
   <213> Artificial
<220>
   <223> primer for the Lactobacillus pentosus
<400> 2
   gcaagtcgaa cgaactctgg tatt 24
<210> 3
   <211> 17
   <212> DNA
   <213> Artificial
<220>
   <223> primer for the Lactobacillus pentosus
<400> 3
   cggaccatgc ggtccaa 17